# EUROPEAN PATENT APPLICATION

(11) **EP 4 759 281 A1**
(43) Date of publication of application: **17.06.2026**
(21) Application number: 25189614.8
(22) Date of filing: 15.07.2025
(51) Int. Cl.: A61F 13/00, A61F 13/02

(54) **SHIELDING DEVICE**

(30) Priority: 12.12.2024 EP 24219385
(71) Applicant: Prosys International Ltd, London SW19 5 AP (GB)
(72) Inventor: STEER, Graham E., London, SW 19 5 AP (GB)
(74) Representative: dompatent

(57) **Abstract**

Shielding device for protecting an application area of a patient's skin comprising: a first upper layer extending along an extension plane being delimited by an outer contour, and a second collar layer extending along an extension plane formed in a ring shape having an outer size and an outer contour identical to the outer contour of first upper layer and having a ring width along the extension plane forming an inner ring contour, wherein the first upper layer and the second collar layer are laid on top of each other with matching outer contours and joined together in the area of their outer contours to form a fluid tight pouch between the first upper layer and the second collar layer, wherein the pouch has a lower opening formed by the inner ring contour of the second collar layer, wherein the second collar layer comprises an adhesive seal on a second surface of the extension plane facing away from the pouch, and wherein the adhesive seal extends circumferential around the inner ring contour and is adapted to removably adhere the shielding device to the patient's skin to surround an application area of the patient and to form a fluid tight enclosure above the application area.

## Description

The present invention is related to a shielding device for protecting an application area of a patient's skin. The intended application area where the shielding device of the current invention can be applied to can be generally a wound site of a patient, all infusion sites for parenteral feeding, oncology fluid infusion sites, all chemotherapy, immunology and hormone therapy PIC and shunt line sites, all vascular access sites for dialysis, all ports under skin, also all areas where the shielding device covers body worn pumps and meter and injectors.

In the field of medical procedures, it is common to use shielding devices such as plasters and/or wound covers to protect the patient's skin and wounds from exposure to the external surrounding and help to prevent that external fluids or particles reach the wound area and maintain a sterile environment. Traditional shielding devices often consist of multiple layers that are joined together to form an inseparable layer stack including a wound dressing on one surface of the shielding device which is adhered to the skin around the application area. These devices typically include an adhesive layer to secure the device to the skin and a protective layer to cover the application area. One major drawback is that the known shielding devices are flat and lack the ability to form an enclosure with a dedicated height above the application area. This limitation makes them unsuitable for shielding ports or other medical devices attached to the patient which require a protective enclosure that extends above the skin surface. As a result, these flat shielding devices fail to provide adequate protection and can lead to contamination of the application area.

Taken aforementioned disadvantages of the known prior art, it is the object of the current invention to overcome the disadvantages of the prior art by providing a shielding device that ensures a fluid-tight seal around the application area, minimizes skin irritation, and is easy to apply and remove. Importantly, the invention addresses the need for a shielding device that can form an enclosure with a dedicated height to protect ports or other medical devices attached to the patient.

The object of the current invention is solved according to the current invention by a shielding device for protecting an application area of a patient's skin. The shielding device comprises a first upper layer extending along an extension plane being delimited by an outer contour and a second collar layer extending along an extension plane and formed in a ring shape along the extension plane having an outer size and outer contour identical to the outer contour of the first upper layer and having a ring width along the extension plane forming an inner ring contour. The first upper layer and the second collar layer are laid on top of each other with matching outer contours and are joined together in the area of their outer contours to form a fluid tight pouch between the first upper layer and the second collar layer, wherein the pouch has a lower opening formed by the inner ring contour of the second collar, wherein the second collar layer comprises an adhesive seal on a second surface of the extension plane facing away from the pouch and wherein the adhesive seal extends circumferentially around the inner ring contour and is adapted to removably adhere the shielding device to the patient's skin to surround an application area of the patient and to form a fluid tight enclosure above the application area.

The extension plane of the first upper layer has a first surface facing away from the pouch and a second opposite surface facing toward the pouch and forming a first partial area of the inner wall of the pouch. The extension plane of the second collar layer has a first surface facing toward the pouch and forming a second partial area of the inner wall of the pouch and a second surface facing away from the pouch. The first upper layer and the second collar layer are each formed by a thin plastic film material wherein the plastic film material is fluid tight. Preferably, the plastic film material of the first upper layer and/or the second collar layer are transparent.

The fluid tight pouch is formed between the second surface of the first upper layer and the first surface of the second collar layer. The pouch being delimited to the outer side by the joint between the first upper layer and the second collar layer in the area of/around their outer contours. The first upper layer and the second collar layer are only joined in the area of the outer contour, otherwise, the two layers are not connected to each other.

When the claimed shielding device is applied to the patient's skin in an application area, the fluid tight enclosure is formed between the skin of the patient and the pouch, wherein the opening of the pouch is sealed to the patient's skin in a fluid tight manner by application of the adhesive seal to the patient's skin to surround the application area/application site. The adhesive seal enables to form with the skin of the patient at the application site an enclosed protective room formed by the pouch. The pouch which is itself formed by and between the first upper shield layer and the second collar layer is able to adapt its inner pouch volume and size during the application of the device to the wound site by unfolding/distancing the first upper layer with respect to the second collar layer.

This shielding device ensures a fluid-tight seal to maintain a sterile environment for the application site and features a secure, removable adhesive for reliable attachment. Its ring-shaped second collar layer allows for versatile application across different application areas and application scenarios, enhancing its adaptability. Overall, it combines robust protection with patient comfort and practical usability.

The outer contour and the size of the first upper layer and the second collar layer are adapted to the specific application site of a patient. The adaption to the application area can be achieved, for example, by an adaption of the size, the geometry, the elasticity of the upper layer and the second collar layer. The outer contours can be formed as square or rectangular with rounded edges, alternatively a round or elliptical outer contour can be foreseen for the layers.

The inner ring counter of the second collar layer in the extension plane can be provided to extend in parallel to the outer contour of the second collar layer, whereby the second collar layer has a consistent ring width. Nevertheless, according to the current invention, it can be also provided that the second collar layer has a varying ring width along the contour so that the inner ring contour does not extend in parallel to the outer ring contour.

The adhesive seal preferably does not cover the whole surface of the second surface but only covers a ring-shaped partial area of the second surface, most preferably neighboured to the inner ring contour of the collar layer. The adhesive seal circumferentially surrounds the inner ring contour and thereby, the lower opening of the pouch of the shielding device.

Preferably the adhesive seal comprises at least in a partial area of the second surface around the inner ring contour, more preferably neighboured to the inner ring contour of the collar layer, a moisture sensitive indicator which is adapted to undergo a colour change when exposed to water. The inclusion of a moisture-sensitive indicator in the adhesive seal enables early, visual detection of fluid ingress at the interface between the device and the patient's skin. This allows timely intervention, enhancing patient safety by reducing the risk of unnoticed leakage or contamination.

The moisture sensitive indicator can comprise a hydrochromic acrylic layer extending circumferentially or in multiple segments around the inner ring contour, preferably neighboured to the inner ring contour of the collar layer, preferably and having a ring width smaller the ring width of the third film layer. The use of a hydrochromic acrylic layer as a moisture-sensitive indicator provides a clear, immediate visual cue upon fluid exposure, improving the reliability of seal integrity monitoring. Its circumferential or segmented arrangement near the inner ring contour ensures targeted detection at the most critical area.

It can be provided that the sensitive indicator when exposed to water undergoes a permanent colour change from initially (when dry) colourless to blue when in exposed to water. The features enabling a clear and unambiguous detection of moisture/water ingress. This irreversible response ensures that even transient fluid/water contact is recorded, supporting safe use in clinical and home care settings by alerting users to compromised seals or unexpected fluid accumulation.

It can be provided that the adhesive seal comprises a third film layer and a carrier layer, wherein the third film layer is formed in a ring shape with a first surface and a second surface, wherein the first surface of the third film layer is joined together with the second surface of the second collar layer along a ring shaped partial area of the first surface, wherein the carrier layer extends along an extension plane and is formed in a ring shape with a first surface facing towards the second surface of the third film layer and an opposite second surface facing away from the third film layer, wherein the first surface of the carrier layer is permanently fixed to the second surface of the third film layer at least in a ring shaped partial fixation area extending continuously around the inner ring contour and wherein the second surface is adapted to removably adhere the shielding device to the patient's skin to surround an application area of the patient and to form a fluid tight enclosure above the application area. The layered construction of the adhesive seal, comprising a third film layer and a carrier layer, enables mechanical decoupling between the sealing function and the skin-contact surface, thereby improving structural stability and adhesive reliability. The permanent fixation of the carrier layer to the third film layer around the inner ring contour ensures a consistent and secure bonding interface, while the removable skin contact surface facilitates safe, fluid-tight, and non-invasive attachment to the patient.

The third film layer can be formed by a plastic material, wherein the plastic material is fluid tight. Preferably, the third film layer is identical in size and shape to the second collar layer. The joint between the third film layer and the second collar layer is fluid tight. It can be provided that the carrier layer having the same size and the same inner contour as well as outer contour as the third film layer. By configuring the carrier layer to have the same size and matching inner and outer contours as the third film layer, the adhesive seal achieves precise layer alignment and uniform mechanical performance across the entire sealing area. This geometric congruence simplifies manufacturing, enhances lamination accuracy, and ensures consistent adhesion and flexibility at the skin-contact interface.

The second surface of the carrier layer can be coated at least in a partial area extending continuously around the inner ring contour with a solvent based water stable acrylic adhesive to removably adhere the shielding device to the patient's skin and to form a fluid tight seal with the patient's skin. Coating the second surface of the carrier layer with a solvent-based, water-stable acrylic adhesive ensures a reliable and durable bond to the patient's skin, even in moist or high-humidity environments. This configuration supports the formation of a fluid-tight seal around the application area while maintaining removability, thereby enhancing both patient comfort and device safety during medical use.

The carrier layer can be formed by a transparent plastic film layer, preferably by a polyethylene terephthalate, PET, film layer. Forming the carrier layer from a transparent plastic film, preferably polyethylene terephthalate (PET), provides high tensile strength, dimensional stability, and excellent transparency for visual monitoring of the underlying skin or indicator areas. PET's chemical resistance and medical-grade compatibility make it ideal for use in adhesive medical devices, supporting both durability and patient safety.

Preferably the first surface of the carrier layer is permanently fixed to the second surface of the third film layer by a glue layer, preferably by a ethylene-acrylic acid copolymer, EAA, glue layer, which is adapted to permanently fix the first surface of the carrier layer to the second surface of the third film layer. Permanently fixing the first surface of the carrier layer to the second surface of the third film layer using a glue layer-preferably an ethylene-acrylic acid (EAA) copolymer-ensures strong, durable lamination with excellent adhesion between dissimilar polymer layers. The EAA glue layer provides chemical compatibility, flexibility, and moisture resistance, thereby enhancing the structural integrity and long-term reliability of the adhesive seal in medical applications.

The moisture indicator is preferably arranged in the area of the second surface of the carrier layer. Positioning the moisture indicator on the second surface of the carrier layer enables direct exposure to potential fluid ingress at the skin interface, ensuring timely and localized detection of seal breaches. This arrangement maximizes indicator sensitivity while maintaining compatibility with the multilayer adhesive structure, without interfering with bonding or comfort.

The third film layer can be formed by an Ethylene Vinyl Acetate, EVA, film, preferably with a thickness between the first and the second surface of 200 micron.

It can be foreseen that the first upper layer and/or the second collar layer is/are formed by a clear Ethylene Vinyl Acetate, EVA, film, preferably with a layer thickness of 75 micron. The EVA film has the advantage of being highly flexible, moisture-resistant, and durable, making it ideal for wound coverings that need to conform to the body and protect wounds from external contaminants. Additionally, its softness and strong adhesion ensures comfort and secure placement, while providing UV protection for exposed wounds.

The layer thickness of each layer is measured as the shortest distance between the respective first and second surface of the specific layer.

Preferably, the adhesive seal is covered by a fourth release liner layer.

The fourth release liner layer can be formed by a siliconized releasable paper liner. The siliconized releasable paper liner protects the adhesive of the device from contamination and ensures easy, clean application. This maintains the adhesive's performance and compliance with regulatory standards, enhancing the device's reliability and effectiveness. The smooth, non-stick surface of a siliconized liner ensures easy and clean application by allowing the liner to be removed without pulling or damaging the adhesive.

The fourth release liner layer has an outer contour extending beyond the outer contour of the second collar layer. The fourth release liner extending beyond the outer contour of the second collar layer enables to form a grasping area of the release liner to easily peel off the release liner from the adhesive seal to uncover the adhesive seal prior to its application to the patient's skin.

More preferably, the fourth release liner comprises an additional tab which protrudes from an extension plane of the fourth release paper liner to the underside of the shielding device and provides a grip area to grasp and peel the fourth release liner from the adhesive.

Preferably, the first upper layer and the second collar layer are joined together via a high frequency welding to form a weld along the outer contour. The third film layer can be joined together with the second collar layer along a ring-shaped partial area via high frequency welding. The weld forms the fluid tight joint between the first upper layer and the second collar layer and thereby creates the fluid tight pouch. High frequency welding creates a strong, durable, and fluid-tight seal between the first upper layer and the second collar layer, ensuring reliable protection. This method also provides precision and efficiency in manufacturing, enhancing the device's overall quality and consistency.

In the following, reference will be made to the attached figures describing exemplary embodiments of the shielding device according to the present invention.
- Fig. 1: shows a perspective view of a first exemplary embodiment of a shielding device according to the current invention;
- Fig. 2: shows a bottom view on the underside of the exemplary shielding device according to Fig. 1;
- Fig. 3: shows a cut view of the exemplary embodiment of the shielding device according to Fig. 1 including an enlarged partial view.

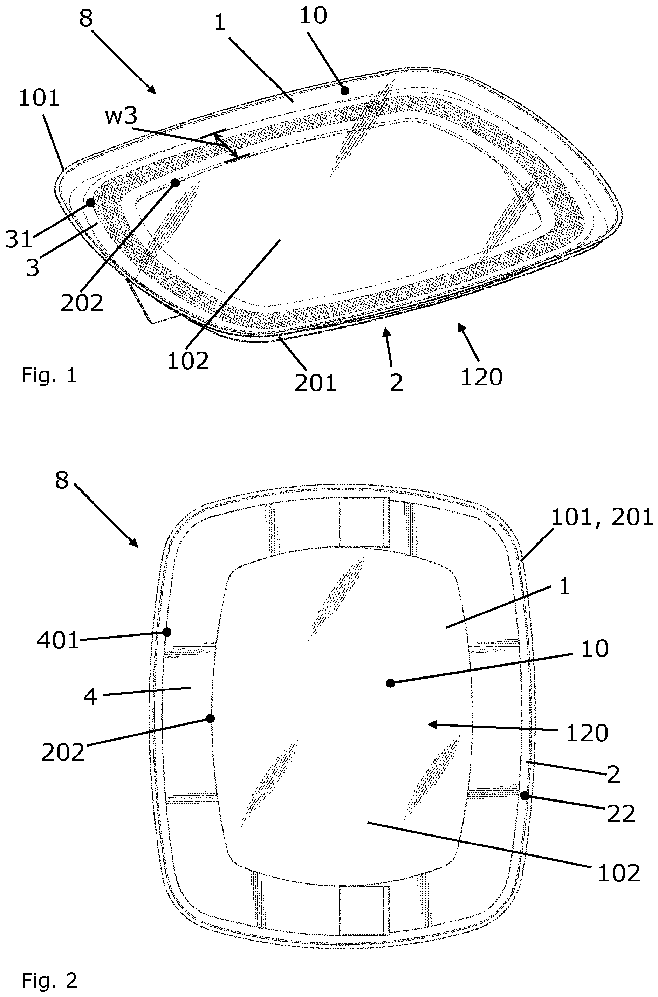

As can be taken from the Figures 1-3, the shielding device 8 adapted to protect an application area of a patient's skin comprises a first upper layer 1 extending along an extension plane 10 and being delimited by an outer contour 101 and a second collar layer 2 extending along an extension plane 20 and formed in a ring shape having an outer size and outer contour 201 identical to the outer contour 101 of the first upper layer 1. The second collar layer 2 has a ring width along the extension plane 20 and forming an inner ring contour 202. The first upper layer 1 and the second collar layer 2 are laid on top of each other with matching outer contours 101, 201 wherein the first upper layer 1 and the second collar layer 2 are joined together in the area of the outer contours 101, 201 of the layers 1 and 2 to form a fluid tight pouch 102 between the first upper layer 1 and the second collar layer 2.

In the exemplary embodiment as shown in the figures, the first upper layer 1 as well as the second collar layer 2 are each formed by a transparent film layer, the layers 1, 2 can be, for example, formed by a clear/transparent Ethylene Vinyl Acetate, EVA film.

The second collar layer 2 comprises an adhesive seal 3 on a second surface 22 of the extension plane 20 facing away from the pouch 102 and wherein the adhesive seal 3 extends circumferentially around the inner ring contour 202 and is adapted to removably adhere the shielding device 8 to the patient's skin to surround an application area of the patient and to form a fluid tight enclosure above the application area.

In the exemplary embodiment as shown in the figures, the adhesive seal 3 is formed by a third film layer (3) and a carrier layer 33, the third film layer 3 is formed in a ring shape with a first surface 31 and a second surface 32 wherein the first surface 31 of the third film layer 3 is joined together with the second surface 22 of the second collar layer 2 along a ring-shaped partial area of the first surface 31, wherein the carrier layer 33 extends along an extension plane and is formed in a ring shape with a first surface 331 facing towards the second surface 32 of the third film layer 3 and an opposite second surface 332 facing away from the third film layer 3, wherein the first surface 331 of the carrier layer 33 is permanently fixed to the second surface 32 of the third film layer 3 at least in a ring shaped partial fixation area extending continuously around the inner ring contour 202 and wherein the second surface 332 is adapted to removably adhere the shielding device 8 to the patient's skin to surround an application area of the patient and to form a fluid tight enclosure above the application area.

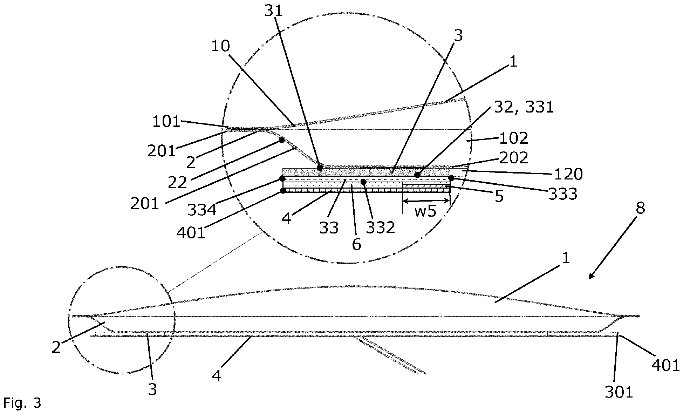

In the exemplary embodiment according to Fig. 3 the second surface 332 of the carrier layer 33 is coated at least in a partial area extending continuously around the inner ring contour 202 with a solvent based water stable acrylic adhesive 6 to removably adhere the shielding device 8 to the patient's skin and to form a fluid tight seal with the patient's skin. Furthermore a moisture indicator 5 is arranged in the area of the second surface 332 of the carrier layer 33 around the inner ring contour 202 which is adapted to undergo a colour change when exposed to water. The moisture sensitive indicator 5 can comprise a hydrochromic acrylic layer extending circumferentially or in multiple segments around the inner ring contour 202 and having a ring width w5 smaller the ring width w3 of the third film layer (3).

The exemplary embodiments according to the Figures 1-4 show an embodiment wherein the third film layer 3 is provided with a smaller outer diameter and a smaller outer contour, thereby being recessed in the extension plane from the outer contour of the second collar layer 2. The third film layer has a ring width w3 along its extension plane. The third film layer 3 can be formed by a transparent plastic film for example from an EVA film.

The exemplary embodiment shows the optional feature that the adhesive seal 3 is covered by a fourth release liner layer 4 wherein the aforementioned release liner layer 4 can be, for example, formed by a siliconized release paper liner.

## Claims

1. Shielding device (8) for protecting an application area of a patient's skin comprising:
a first upper layer (1) extending along an extension plane (10) being delimited by an outer contour (101), and
a second collar layer (2) extending along an extension plane (20) formed in a ring shape having an outer size and an outer contour (201) identical to the outer contour (101) of first upper layer (1) and having a ring width (w2) along the extension plane (20) forming an inner ring contour (202),
wherein the first upper layer (1) and the second collar layer (2) are laid on top of each other with matching outer contours (101, 201) and joined together in the area of their outer contours (101, 201) to form a fluid tight pouch (102) between the first upper layer (1) and the second collar layer (2);
wherein the pouch (102) has a lower opening (120) formed by the inner ring contour (202) of the second collar layer (2);
wherein the second collar layer (2) comprises an adhesive seal (3) on a second surface (22) of the extension plane (20) facing away from the pouch (102); and
wherein the adhesive seal (3) extends circumferential around the inner ring contour (202) and is adapted to removably adhere the shielding device (8) to the patient's skin to surround an application area of the patient and to form a fluid tight enclosure above the application area.

2. Shielding device (8) according to claim 1, wherein the adhesive seal (3) comprises at least in a partial area of the second surface (22) around the inner ring contour (202) a moisture sensitive indicator (5) which is adapted to undergo a colour change when exposed to water.

3. Shielding device (8) according to claim 2, wherein the moisture sensitive indicator (5) comprises a hydrochromic acrylic layer extending circumferentially or in multiple segments around the inner ring contour (202) and having a ring width (w5) smaller the ring width (w3) of the third film layer (3).

4. Shielding device (8) according any one of claims 1 to 3, wherein the adhesive seal (3) comprises a third film layer (3) and a carrier layer (33), wherein the third film layer (3) is formed in a ring shape with a first surface (31) and a second surface (32), wherein the first surface (31) of the third film layer (3) is joined together with the second surface (22) of the second collar layer (2) along a ring shaped partial area of the first surface (31), wherein the carrier layer (33) extends along an extension plane (30) and is formed in a ring shape with a first surface (331) facing towards the second surface (32) of the third film layer (3) and an opposite second surface (332) facing away from the third film layer (3), wherein the first surface (331) of the carrier layer (33) is permanently fixed to the second surface (32) of the third film layer (3) at least in a ring shaped partial fixation area extending continuously around the inner ring contour (202) and wherein the second surface (332) is adapted to removably adhere the shielding device (8) to the patient's skin to surround an application area of the patient and to form a fluid tight enclosure above the application area.

5. Shielding device (8) according to claim 4, wherein the carrier layer (33) having the same size and the same inner contour (333) as well as outer contour (334) as the third film layer (3).

6. Shielding device (8) according to any one of claims 4 or 5, wherein the second surface (332) of the carrier layer (33) is coated at least in a partial area extending continuously around the inner ring contour (202) with a solvent based water stable acrylic adhesive (6) to removably adhere the shielding device (8) to the patient's skin and to form a fluid tight seal with the patient's skin.

7. Shielding device (8) according to anyone of claims 4 to 6, wherein the carrier layer (33) is formed by a transparent plastic film layer, preferably by a polyethylene terephthalate, PET, film layer.

8. Shielding device (8) according to anyone of claims 4 to 7, wherein the first surface (331) of the carrier layer (33) is permanently fixed to the second surface (32) of the third film layer (3) by a glue layer, preferably by a ethylene-acrylic acid copolymer, EAA, glue layer, which is adapted to permanently fix the first surface (331) of the carrier layer (33) to the second surface (32) of the third film layer (3).

9. Shielding device (8) according to anyone of claims 4 to 8, comprising a moisture indicator (5) according to claims 2 or 3, wherein the moisture indicator (5) is arranged in the area of the second surface (332) of the carrier layer (33).

10. Shielding device (8) according to any one of the preceding claims, wherein the first upper layer (1) and/or the second collar layer (2) is/are formed by a clear Ethylene Vinyl Acetate, EVA, film, preferably with a layer thickness of 75 micron.

11. Shielding device (8) according to any one of the claims 4 to 10, wherein the third film layer (3) is formed by a clear Ethylene Vinyl Acetate, EVA, film, preferably with a layer thickness of 200 micron.

12. Shielding device (8) according to any one of the preceding claims, wherein the adhesive seal (3) is covered by a fourth release liner layer (4).

13. Shielding device (8) according to claim 10, wherein the fourth release liner layer (4) is formed by a siliconized releasable paper liner (4).

14. Shielding device (8) according to any one of the preceding claims, wherein the first upper layer (1) and the second collar layer (2) are joined together via high frequency welding to form a weld along the outer contour (101, 201).

15. Shielding device (8) according to any one of the claims 4 to 14, wherein the third film layer (3) is joined together with the second collar layer (2) along a ring-shaped partial area via high frequency welding.
